# EUROPÄISCHE PATENTSCHRIFT

(11) **EP 1 478 749 B1**
(45) Veröffentlichungstag und Bekanntmachung des Hinweises auf die Patenterteilung: **14.04.2010**
(21) Anmeldenummer: 03706546.3
(22) Anmeldetag: 20.02.2003
(51) Int. Cl.: C12N 15/10

(54) **VERFAHREN ZUR GROBKLÄRUNG VON ZELLAUFSCHLÜSSEN AUS MIKROORGANISMEN**
METHOD FOR COARSE PURIFICATION OF CELL DIGESTS FROM MICROORGANISMS
PROCEDE POUR LA PURIFICATION GROSSIERE DE LYSATS CELLULAIRES PROVENANT DE MICRO-ORGANISMES

(30) Priorität: 20.02.2002 DE 10207170
(43) Veröffentlichungstag der Anmeldung: 24.11.2004
(73) Patentinhaber: QIAGEN GmbH, 40724 Hilden (DE)
(72) Erfinder: HUCKLENBROICH, Jörg, 42857 Remscheid (DE); MÜLLER, Markus, 41542 Dormagen (DE)
(86) Internationale Anmeldenummer: PCT/EP2003/001769
(87) Internationale Veröffentlichungsnummer: WO 2003/070942

(56) Entgegenhaltungen:
- EP-A- 0 875 271
- US-B1- 6 218 531
- THOMAS CHRISTOPHER M: "Paradigms of plasmid organization." MOLECULAR MICROBIOLOGY, Bd. 37, Nr. 3, August 2000 (2000-08), Seiten 485-491, XP002242117 ISSN: 0950-382X
- CONN GRAEME L ET AL: "RNA structure." CURRENT OPINION IN STRUCTURAL BIOLOGY, Bd. 8, Nr. 3, Juni 1998 (1998-06), Seiten 278-285, XP002242118 ISSN: 0959-440X

## Beschreibung

Die vorliegende Erfindung betrifft ein Verfahren zur Grobklärung von Zellaufschlüssen von Mikroorganismen, insbesondere von Bakterien - wie zum Beispiel *E.coli*.

Molekularbiologische Verfahren gewinnen zunehmend an Bedeutung in einer-Vielzahl von Anwendungsgebieten - wie z.B. insbesondere bei der Herstellung von Arzneimitteln. Bei der Gewinnung von sog. Plasmid-DNA stellt sich insbesondere das Problem, die Plasmid-DNA in möglichst reiner Form zu gewinnen. Um beispielsweise Plasmid-DNA im präparativen Maßstab herstellen zu können, ist es erforderlich, die Plasmide mit Hilfe von sog. Wirtszellen zu vermehren. Hierbei handelt es sich im allgemeinen um gram-positive oder aber auch gram-negative Bakterien - wie z.B. Mutanten von *E.coli*.

Ein besonderes Problem stellt in diesem Zusammenhang das Abtrennen von unerwünschter genomischer DNA, RNA und Endotoxinen dar, die aus der für die Vermehrung eingesetzten Mikroorganismen stammen. Da die genannten Nukleinsäuren zur selben Klasse von Biomolekülen gehören, haben sie auch bestimmte vergleichbare physikochemische Eigenschaften, wie Plasmid-DNA und stellen somit eine enorme Schwierigkeit bei der Aufreinigung bzw. Abtrennung der Plasmid-DNA dar.

Während das Abtrennen der unerwünschten bzw. kontaminierenden Nukleinsäuren und Endotoxine im analytischen Maßstab ein beherrschbares Problem darstellt, kann dies im präparativen Maßstab bislang nur mit einem wesentlich höheren Aufwand mit vergleichbaren Erfolg erzielt werden. Bei der Lösung derartiger Aufreinigungsprobleme ist man in der überwiegenden Mehrzahl der Fälle bestrebt, das Abtrennen von unerwünschten Verunreinigungen auf einer möglichst frühen Prozessstufe durchzuführen, um ein Verschleppen der Verunreinigungen zu vermeiden.

Der Zellaufschluss von Mikroorganismen stellt den initialen Schritt bei der Isolierung bzw. Reinigung von Plasmid-DNA aus derartigen Mikroorganismen dar. Nach der Durchführung der Lyse - die üblicherweise mittels eines Lysepuffers wie z.B. SDS (Natrium-Dodecylsulfat) in Gegenwart von Natriumhydroxid erfolgt - liegen neben den einzelnen Zellbestandteilen auch die Bruchstücke der Zellwand in der Reaktionslösung vor, an die die genomische DNA über Membran-assoziierte Proteine gebunden ist. Diese Bindung ist allerdings recht schwach ausgebildet, so dass mechanische Einwirkungen ausreichen, um diese Bindungen aufzulösen. Die so in den wässrigen Überstand freigesetzte genomische DNA ist dann nicht mehr auf einfachem Wege von der Plasmid-DNA oder RNA in späteren Reinigungsschritten abzutrennen.

Bei der Herstellung von Zellaufschlüssen kommt es während des Aufarbeitungsprozesses nach der Neutralisation bzw. Ansäuerung der aus der oben erwähnten basischen Lyse resultierenden Lösung - beispielsweise mittels wässriger Kaliumacteat-Lösung - zur Bildung von flockulären Präzipitaten. Diese Präzipitate bestehen aus Kalium-Dodecylsulfat (KDS, dem Kaliumsalz von SDS) welches durch Anlagerung und Denaturierung an bzw. von Zellwand-assoziierten Proteinen zusammen mit der Zellwand und den Zellmembranen und anderen Zellbestandteilen ausfällt. In diesen Komplexen ist somit auch die an der Zellwand verankerte genomische DNA gebunden. Bei kleineren - beispielsweise analytischen - Präparationen kann die Abtrennung des KDS-Protein-Zellwand-Komplexes auf dem Wege der Zentrifugation erfolgen, wodurch gleichzeitig auch die genomische DNA weitestgehend entfernt wird.

EP-A-0 875 271 beschreibt ein Verfahren, bei dem unter Druckdifferenz eine Mischung durch einen Filter gesaugt wird, um dort eine mechanische Trennung der Mischungsbestandteile zu erreichen.

US-B1-6;218,531 betrifft ein Verfahren zur Lyse (Aufbrechen) der Zellwände von Biomaterial, welches dann auf konventionelle Methoden weiterverarbeitet wird. Es ist kein Verfahren zur Grobklärung eines bereits lysierten Zellmaterials.

Im Hinblick auf sog. "High-Throughput"-Applikationen oder Präparationen in größere Maßstäben ist die Separation des flockulären Präzipitates vom Überstand durch Zentrifugation oder Filtration durch die oben erwähnten Hindernisse nicht sinnvoll oder mit erheblichen Problemen behaftet. So ist die Zentrifugation z.B. im "Batch" nur in einem sehr beschränkten Maß auf größere Ansätze übertragbar, während eine kontinuierliche Zentrifugation den Anteil an aus dem KDS-Präzipitat freigesetzter genomischer DNA im wässrigen Überstand erhöht.

Bei der Filtration ergeben sich im präparativen Maßstab Verblockungs- und Scherprobleme während der Beladung des Filters mit Flocken-belasteten Bakterienlysates. Auch hierdurch würde der Gehalt an genomischer DNA im Filtrat die Plasmid - Phase stark kontaminieren. Als weiterer Nachteil kommt daneben häufig ein Verstopfen des Filters dazu.

Die Aufgabe der vorliegenden Erfindung besteht somit vornehmlich darin, ein Verfahren zur Gewinnung von Plasmid-DNA oder RNA zur Verfügung zu stellen, das im präparativen Maßstab anwendbar ist und bei dem die Plasmid-DNA oder RNAim Ergebnis weitgehend frei von genomischer DNA und Endotoxinen gewonnen werden kann.

Eine weitere Aufgabe der vorliegenden Erfindung besteht darin, ein möglichst einfaches und praktikables Verfahren zur Verfügung zu stellen, das die aus dem Stand der Technik bekannten Nachteile vermeidet.

Gelöst werden die oben genannten Aufgaben dadurch, dass man einen Zellaufschluss biologischen Materials (Mikroorganismen) auf an sich aus dem Stand der Technik bekannte Art und Weise herstellt. Die resultierende Reaktionsmischung wird einer Druckänderung, d.h. einer Druckerhöhung oder -verminderung gegenüber dem Normaldruck, wobei eine Druckverminderung bevorzugt wird, unterworfen.

Die vorliegende Erfindung betrifft somit ein Verfahren zur Grobklärung von Zellaufschlüssen aus Mikroorganismen, dadurch gekennzeichnet, dass das aus dem Zellaufschluss resultierende Lysat einem verminderten oder erhöhtem Druck bezogen auf Normaldruck unterworfen wird, was zu einem Aufschwimmen oder Absinken der Flocken führt und anschließend die flockuläre Phase von der flüssigen Phase separiert wird.

Die Unteransprüche betreffen bevorzugte Ausführungsformen des Verfahrens.

Der überraschende Vorteil dieses Vertahrensschritts besteht darin, dass durch die erfindungsgemäße Änderung des Drucks der die Reaktionsmischung umgebenden Atmosphäre zu einem Aufschwimmen oder Absinken der Flocken führt was letztendlich in einer räumlich stark komprimierten flockulären Phase resultiert. Dabei setzt sich die flockuläre Phase auf der flüssigen Phase ab und lässt sich sehr gut und vollständig von der flüssigen Phase separieren. Bei dem erfindungsgemäß vorgeschlagenen Verfahren wird vorzugsweise der Druck, der die Reaktionsmischung umgebenden Atmosphäre auf einen Wert eingestellt, der 200 bis 1000 mbar niedriger ist als der Normaldruck (Luftdruck unter Normalbedingungen). Besonders bevorzugt wird eine Reduktion des Drucks um einen Wert in einem Intervall von 300 bis 800 mbar.

Ein ähnlicher Effekt stellt sich bei der Wahl eines Drucks oberhalb des Umgebungsdrucks - vorzugsweise von 200 bis 5000 mbar und besonders bevorzugt von 200 bis 2500 mbar- über dem Normaldruck ein. Durch diese Maßnahme wird ebenfalls die Ausbildung einer flockulären Phase erreicht, die in diesem Fall jedoch nicht auf der Flüssigkeit schwimmt sondern absinkt.

Durch die erfindungsgemäße Verfahrensmaßnahme wird somit folgendes zusammenfassend bewirkt:
1. Die Phasentrennung nimmt - auch bei größeren Volumina von 50 Litern und mehr - nur einen Zeitraum von wenigen Minuten in Anspruch und nicht mehr als 30 bis 180 Minuten.
2. Die Phasentrennung führt zu einer räumlich stark komprimierten flockulären Phase, womit sich im gleichen Zug der Volumenanteil der Produkt-haltigen wässrigen Phase vergrößert.

Diese überraschenden Effekte beinhalten insbesondere folgende Vorteile:
1. Es werden keine KDS-Flocken bei nachfolgenden Filtrations- oder Zentrifugationsschritten Scherkräften ausgesetzt.
2. Beim Einsatz größerer Mengen lassen sich die Prozesszeiten für die Klärung von Zellaufschlüssen - wie z.B. in der Plasmid DNA Produktion unter Verwendung von *E.coli -* signifikant verringern, da die eingesetzten Filtrationseinheiten nicht mehr oder langsamer verblocken, sowie höhere Durchflussraten gewählt werden können. Die Ausbeute lässt sich mit dem erfindungsgemäßen Verfahren um ca. 10 bis 30 % erhöhen (da die das Flockulat beinhaltende Phase deutlich komprimierter ist als die entsprechende Phase, die unter Normaldruck-Bedingung hergestellt werden kann).
3. Bei automatisierten "High-Throughput"- Anwendungen - bzw. Anwendungen im sog. "bench-scale" - kann durch Anwendung des erfindungsgemäßen Verfahrens auf zeitaufwendige Zentrifugationsschritte verzichtet werden bzw. die Filtration kann durch die erfindungsgemäß erzielbare Vorseparierung der flockulären Phase von der wässrigen Phase beschleunigt und die Gefahr z.B. des Verstopfens der Filtermembran deutlich reduziert werden. Diese Technologie bietet sich des weiteren dafür an, in vollständig automatisierten "High-Throughput"-Anwendungen integriert zu werden.
4. Die erfindungsgemäß erzielbare Kompaktierung des Flockulats erfolgt extrem scherungsarm, womit - wie oben erwähnt - die Gefahr der Ablösung der genomischen DNA und Endotoxine von den Bruchstücken der Zellwand auf ein Minimum reduziert wird.

Besonders der letzte beschriebene Effekt erweist sich als außerordentlich vorteilhaft, da mittels des erfindungsgemäßen Verfahrens eine Plasmid DNA oder RNA gewonnen werden kann, die mit einem vernachlässigbar geringen Anteil an genomischer DNA und Endotoxinen kontaminiert ist. Ähnliche vorteilhafte Effekte waren aus dem Stand der Technik bislang nur für Verfahren bekannt, bei denen die Separierungen z.B. auf dem Wege der Sedimentation erzielt wurde; es liegt jedoch für den Fachmann auf der Hand, dass derartige Separierungen durch Sedimentation außerordentlich zeitaufwendige Verfahrensschritte verkörpern, die - im Falle einer unvollständigen Phasentrennung - zu einem unbefriedigenden Ergebnis führen und die nicht unbegrenzt in den industriellen Produktionsmaßstab hochskaliert werden können. Bei der Behandlung größerer Ansätze scheidet dieses Verfahren daher unzweifelhaft auf jeden Fall als ernsthaft anwendbare Alternative aus.

Ein weiterer Vorteil des erfindungsgemäßen Verfahrens besteht darin, dass bei nachgeschalteten Filtrationsschritten die Filter deutlich langsamer verblocken als bei dem Einsatz eines ungeklärten Bakterien-Lysats.

Zur Durchführung der erfindungsgemäßen Gewinnung von Plasmid-DNA und RNA aus Zellen von Mikroorganismen wird das Ausgangsmaterial einer der aus dem Stand der Technik bekannten alkalischen Lyse unterworfen.

Nach einer ggf. erforderlichen Neutralisation des aus dem Lyseschritt resultierenden Gemisches wird ein Vakuum mit einem Druck, der 200 bis 1000 mbar niedriger ist als der Normaldruck (Luftdruck), angelegt. Besonders bevorzugt wird eine Reduktion des Drucks um einen Wert in einem Intervall von 300 bis 800 mbar.

In einer alternativen Ausführungsform wird ein Umgebungsdruck oberhalb des Normaldruck gewählt - vorzugsweise in einem Intervall von 200 bis 5000 mbar und besonders bevorzugt in einem Intervall von 200 bis 2500 mbar - über dem Normaldruck.

### Erläuterung der Abbildungen:

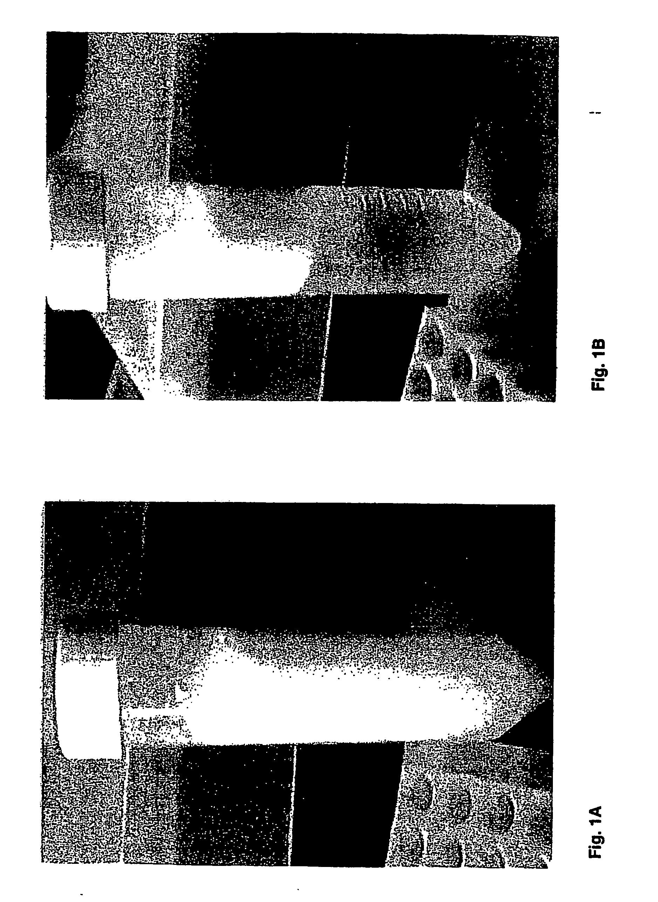

Fig. 1 zeigt ein Reaktionsgemisch vor der Anwendung des erfindungsgemäßen Verfahrens (Fig. 1A) sowie nach dem Anlegen eines Vakuums (Fig. 1 B) im 50 ml-Maßstab.

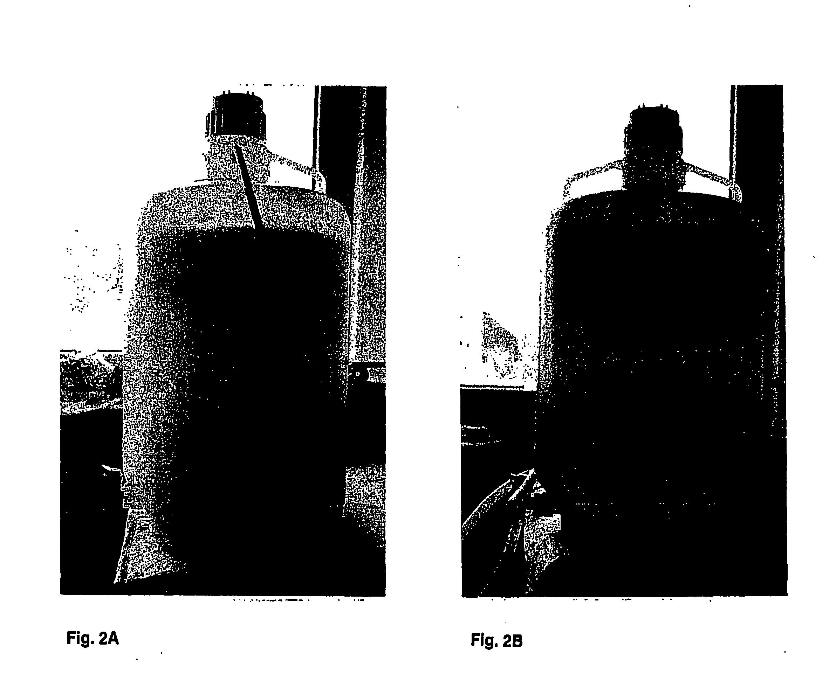

Fig. 2 zeigt einen Lyse-Ansatz gemäß Beispiel 3 in einem 50-Liter-Maßstab - wie in Fig. 1 - vor dem Anlegen eines Vakuums (Fig. 2A) sowie nach der Anwendung des erfindungsgemäßen Verfahrens (Fig. 2B).

Die nachfolgenden Beispiele sollen die Erfindung erläutern:

### Beispiel 1

68 g gefrorene pUC19 Biomasse werden in einem Becherglas und in einem Liter eines handelsüblichen Resuspendierungspuffers (P1 Puffer der Fa. QIAGEN, D-40724 Hilden) aufgetaut und durch Schütteln homogenisiert. Danach wird 1 Liter eines handelsüblichen Zell-Lysepuffers zugefügt (P2 Puffer der Fa. QIAGEN, D-40724 Hilden) und die Biomasse der Lyse unterworfen. Dabei wird der Lyseprozess z.B. durch Schütteln unterstützt. Die Inkubationszeit für diesen Schritt beträgt mindestens 5 Minuten. Das so erhaltene Lysat wird in ein geeignet großes Reaktionsgefäß zum Abklären überführt (Volumen ca. 5 Liter) und mit einem Liter eines eisgekühltem handelsüblichen Neutralisationspuffers (P3 Puffer der Fa. QIAGEN, D-40724 Hilden) vermischt. Danach wird an das Reaktionsgefäß ein leichtes Vakuum von ca. 500 mbar unter Normaldruck über einen Zeitraum von ca. 3 Minuten angelegt. Dabei ist ein sofortiges Aufschwimmen der festen Bestandteile zu beobachten. Das in der unteren Phase befindliche, geklärte Lysat ist frei von festen Bestandteilen (gemäß visueller Prüfung) und kann isoliert bzw. weiter prozessiert werden.

### Beispiel 2

1 g gefrorene pQ73 Biomasse werden in 15 ml eines handelsüblichen Resuspendierungspuffers (P1 Puffer der Fa. QIAGEN, D-40724 Hilden) aufgetaut und resuspendiert. Danach werden 15 ml eines handelsüblichen Zell-Lysepuffers (P2 Puffer der Fa. QIAGEN, D-40724 Hilden) zugefügt und die Biomasse der Lyse unterworfen und über einen Zeitraum von ca. 5 Minuten auf Eis inkubiert. Das resultierende Lysat wird mit 15 ml eines eisgekühltem handelsüblichen Neutralisationspuffers (P3 Puffer der Fa. QIAGEN, D-40724 Hilden) vermischt und geschüttelt. Das Reaktionsgefäß wird in einem Exsikkator überführt und über einen Zeitraum ca. 7 Minuten einem Vakuum von 0.7 bar ausgesetzt. Dabei ist ebenfalls ein sofortiges Aufschwimmen der festen Bestandteile zu beobachten.

### Beispiel 3

100 g geerntete Biomasse aus einer 200 Liter Fermentation mit *E.coli,* welches mit dem Plasmid pQ81 transformiert war, werden in 500 ml eines handelsüblichen Resuspendierungspuffers (P1 Puffer der Fa. QIAGEN, D-40724 Hilden) aufgetaut, mit einem Magnetrührstab resuspendiert und in einer 5 Liter Flasche mit 1000 ml Resuspendierungspuffer (P1 Puffer der Fa. QIAGEN, D-40724 Hilden) versetzt. Danach werden 1,5 Liter Zell-Lysepuffer (P2 Puffer der Fa. QIAGEN, D-40724 Hilden) zugegeben und die Suspension durch mehrmaliges Invertieren vermischt. Darauf erfolgt eine Inkubation von 10 Minuten bei Raumtemperatur, während die Bakterien lysieren. Nach der Inkubation werden 1,5 Liter auf 4 - 8 °C eines eisgekühltem handelsüblichen Neutralisationspuffers (P3 Puffer der Fa. QIAGEN, D-40724 Hilden) zugegeben und erneut durch Invertieren vermischt, wobei das Kalium-Dodecylsulfat-Präzipitat (KDS-Präzipitat) ausfällt.

Die so hergestellten Zell-Lysate (10 x 4.5 Liter) werden vorsichtig in eine 50 Liter Abklärflasche dekantiert. Anschließend wird der Flascheninnendruck auf einen Umgebungsdruck von 600 mbar (entspricht -400 mbar gegenüber Normaldruck) reduziert. Das Vakuum wird solange aufrechterhalten, bis alle Flocken an der Oberfläche flotieren. Der Vorgang erfordert ca. 5 - 10 Minuten. Danach wird das Vakuum aufgehoben und das klare Zell-Lysat von unten aus der Abklärflasche abgelassen, bis die oben flokkulierende KDS-Phase den unteren Auslasshahn erreichte. Die so erhaltene flüssige Phase ist gemäß visueller Prüfung frei von sichtbaren Flocken.

### Beispiel 4

1 Liter LB Miller wir mit dem Stamm *E.coli* DH5 alpha RCB angeimpft und ca. 12 h bei einer Temperatur von 37°C in einem Schüttler bebrütet. Danach werden die Bakterien abzentrifugiert. Das so gewonnene Pellet wird in 75 ml eines handelüblichen Resuspendierungspuffers ohne RNase (z.B. P1 Puffer der Firma QIAGEN, D-40724 Hilden) resuspendiert und mit 75 ml eines handelsüblichen Lyse Puffers (z.B. P2 Puffer der Firma QIAGEN, D-40724 Hilden) zugegeben und durch vorsichtiges Schütteln gemischt. Nach erfolgter Lyse der Bakterien wird mit 75 ml eines handelsüblichen Neutralisationspuffers (z.B. P3 Puffer der Firma QIAGEN, D-40724 Hilden) neutralisiert. Das Reaktionsgemisch wird über einen Zeitraum von 2 Minuten einem von gegenüber dem Normaldruck um 700 mbar verminderten Druck ausgesetzt. Dabei findet umgehend eine Absonderung der Zelltrümmer statt, die auf der Flüssigkeitsoberfläche einen kompakten Pfropf ausbilden.

Die nachfolgende Reinigung, z.B. säulenchromatographische Reinigung über die Schritte: Binden der RNA an die Matrix, Waschen der gebundenen RNA mit einem geeigneten Waschpuffer und nachfolgende Elution mit nachfolgender Fällung der RNA aus dem Eluat mit Isopropanol, liefert die RNA in einer Gesamtausbeute von 720 µg RNA guter Qualität.

## Patentansprüche

1. Verfahren zur Grobklärung von Zellaufschlüssen aus Mikroorganismen, **dadurch gekennzeichnet, dass** das aus dem Zellaufschluss resultierende Lysat einem verminderten oder erhöhtem Druck bezogen auf Normaldruck unterworfen wird, was zu einem Aufschwimmen oder Absinken der Flocken führt und anschließend die flockuläre Phase von der flüssigen Phase separiert wird.

2. Verfahren nach Anspruch 1, **dadurch gekennzeichnet, dass** es sich bei dem Mikroorganismus um ein gram-positives oder gram-negatives Bakterium handelt

3. Verfahren nach Anspruch 1 oder 2, **dadurch gekennzeichnet, dass** der Druck der die Reaktionsmischung umgebenden Atmosphäre auf einen Wert eingestellt wird, der um 200 bis 1000 mbar niedriger ist, als der Normaldruck.

4. Verfahren nach Anspruch 3, **dadurch gekennzeichnet, dass** der Druck der die Reaktionsmischung umgebenden Atmosphäre auf einen Wert eingestellt wird, der um 300 bis 800 mbar niedriger ist, als der Normaldruck.

5. Verfahren nach Anspruch 1 oder 2, **dadurch gekennzeichnet, dass** der Druck der die Reaktionsmischung umgebenden Atmosphäre auf einen Wert eingestellt wird, der um 200 bis 5000 mbar höher ist, als der Normaldruck.

6. Verfahren nach Anspruch 5, **dadurch gekennzeichnet, dass** der Druck der die Reaktionsmischung umgebenden Atmosphäre auf einen Wert eingestellt wird, der um 500 bis 2500 mbar höher ist, als der Normaldruck.

## Claims

1. Method of coarsely clarifying cell lysates from microorganisms, **characterized in that** the lysate resulting from the cell lysis is subjected to reduced or increased pressure, in relation to normal pressure, which leads to the flakes floating or sinking, and the flocculent phase is subsequently separated from the liquid phase.

2. Method according to Claim 1, **characterized in that** the microorganism is a gram-positive or gram-negative bacterium.

3. Method according to Claim 1 or 2, **characterized in that** the pressure of the atmosphere surrounding the reaction mixture is adjusted to a level which is 200 to 1000 mbar lower than normal pressure.

4. Method according to Claim 3, **characterized in that** the pressure of the atmosphere surrounding the reaction mixture is adjusted to a level which is 300 to 800 mbar lower than normal pressure.

5. Method according to Claim 1 or 2, **characterized in that** the pressure of the atmosphere surrounding the reaction mixture is adjusted to a level which is 200 to 5000 mbar higher than normal pressure.

6. Method according to Claim 5, **characterized in that** the pressure of the atmosphere surrounding the reaction mixture is adjusted to a level which is 500 to 2500 mbar higher than normal pressure.

## Revendications

1. Procédé pour la purification grossière de digestions cellulaires provenant de microorganismes, **caractérisé en ce que** le lysat obtenu de la digestion cellulaire est soumis à une pression réduite ou augmentée par rapport à la pression normale, ce qui conduit à une flottaison ou une sédimentation des flocons, puis la phase floculée est séparée de la phase liquide.

2. Procédé selon la revendication 1, **caractérisé en ce qu'**il s'agit, pour le microorganisme, d'une bactérie gram-positive ou gram-négative.

3. Procédé selon la revendication 1 ou 2, **caractérisé en ce que** la pression de l'atmosphère entourant le mélange réactionnel est réglée à une valeur qui est inférieure de 200 à 1000 mbars à la pression normale.

4. Procédé selon la revendication 3, **caractérisé en ce que** la pression de l'atmosphère entourant le mélange réactionnel est réglée à une valeur qui est inférieure de 300 à 800 mbars à la pression normale.

5. Procédé selon la revendication 1 ou 2, **caractérisé en ce que** la pression de l'atmosphère entourant le mélange réactionnel est réglée à une valeur qui est supérieure de 200 à 5000 mbars à la pression normale.

6. Procédé selon la revendication 5, **caractérisé en ce que** la pression de l'atmosphère entourant le mélange réactionnel est réglée à une valeur qui est supérieure de 500 à 2500 mbars à la pression normale.
